# EUROPEAN PATENT SPECIFICATION

(11) **EP 2 747 814 B1**
(45) Date of publication and mention of the grant of the patent: **12.08.2020**
(21) Application number: 12808478.7
(22) Date of filing: 22.10.2012
(51) Int. Cl.: A61M 15/00

(54) **SYSTEMS AND METHODS FOR COMBINED RESPIRATORY THERAPY AND RESPIRATORY MONITORING**
SYSTEME UND VERFAHREN FÜR KOMBINIERTE ATEMTHERAPIE UND ATEMÜBERWACHUNG
SYSTÈMES ET PROCÉDÉS DE THÉRAPIE RESPIRATOIRE ET SURVEILLANCE RESPIRATOIRE COMBINÉES

(30) Priority: 27.10.2011 US 201161552096 P
(43) Date of publication of application: 02.07.2014
(73) Proprietor: Koninklijke Philips N.V., 5656 AG Eindhoven (NL)
(72) Inventor: VINK, Teunis Johannes, NL-5656 AE Eindhoven (NL); DERKX, Rene Martinus Marina, NL-5656 AE Eindhoven (NL); FAZZI, Alberto, NL-5656 AE Eindhoven (NL); GEERLINGS, Alexander Cornelis, NL-5656 AE Eindhoven (NL); JANSE, Cornelis Pieter, NL-5656 AE Eindhoven (NL); VAN DE LAAR, Jakob, NL-5656 AE Eindhoven (NL); WALKER, David Paul, NL-5656 AE Eindhoven (NL)
(74) Representative: Philips Intellectual Property & Standards
(86) International application number: PCT/IB2012/055800
(87) International publication number: WO 2013/061240

(56) References cited:
- EP-A2- 1 120 124
- WO-A1-2005/099798
- WO-A1-2010/091462
- WO-A1-2011/083377
- WO-A2-2010/023591
- US-A1- 2003 205 229

## Description

The present disclosure pertains to systems and methods for combined respiratory therapy and respiratory monitoring.

Respiratory diseases are often managed using respiratory therapy devices. For example, drug delivery devices such as metered dose inhalers (MDIs) and other drug delivery devices may be used to provide treatment/therapy to the respiratory system of a patient. MDI's and other drug delivery devices (e.g., dry powdered inhalers, nebulizers, etc.) can be combined with spacers, valved chambers or other implements to help patients (e.g., children) to coordinate the inhalation of delivered drug. Other respiratory therapy devices include ventilators, positive airway pressure (PAP) devices, and/or other devices that provide a flow of gas or pressure to the respiratory system of a patient. Often times, a mask or other patient interface is also used to facilitate respiratory therapy.

Respiratory disease treatment and management also often involves monitoring of lung function. This is sometimes accomplished using peak flow meters (PFMs) to detect upcoming adverse events (exacerbations), assess patient condition, assess disease status, and/or assess treatment/management progression. However, PFMs require patient cooperation, thus, their usefulness is dependent on patient effort. Accordingly, PFMs and other effort dependent monitoring methods are not advantageous for use with children.

Monitoring of lung sounds may provide an effort independent method for lung monitoring. However, complete treatment and monitoring even with the use of lung sound monitoring, is made more difficult because respiratory therapy devices (e.g., drug sources, ventilators, etc.) are completely separate from sound-based lung monitoring systems. Accordingly, system setup can be unwieldy and problematic, especially for use with children. Furthermore, separate systems lead to patients being asked to separately perform monitoring protocols on top of treatment protocols. This creates additional effort for the patient that might lead to a lack of compliance for either or both of the prescribed protocols. Additional difficulties include those related to coordination of lung sound events with treatment events. Separate systems also increase costs, setup and removal time, patient discomfort, and/or present other difficulties.

These and other problems exist.

Examples of background art include: WO 2011/083377 A1 which describes a respiratory drug delivery device that provides feedback regarding the use of the respiratory drug delivery system based on the output of one or more sensors. US 2003/205229 A1 describes a drug delivery apparatus that comprises an indicator that indicates to the patient when a desired dose has been delivered. WO 2010/023591 A2 describes a respiratory drug system with a sound generator adapted to generate one or more audible instructions in response to an actuation signal. WO 2010/091462 A1 describes acoustic detection for automated devices such as respiratory treatment apparatuses. WO2005/099789 A1 describes a method and apparatus for treatment of a user's sleeping disorder, e.g., snoring. EP 1120124 A2 describes a ventilator for respiratory care devised to act on at least one function in the ventilator on the basis of sounds identified by a sound detector.

Accordingly, it is an object of one or more embodiments of the present invention to provide an apparatus for combined respiratory therapy and respiratory monitoring comprising: a spacer portion having first and second ends, the first end having a patient interface configured to engage a respiratory system of a patient; at least one respiratory therapy device connected to the second end of the spacer portion; and a monitoring portion connected to the spacer portion configured to monitor sounds from within the apparatus.

It is yet another aspect of one or more embodiments of the present invention to provide a method for providing combined respiratory therapy and respiratory monitoring, comprising: engaging a combined respiratory therapy and monitoring apparatus to a respiratory system of a patient, the apparatus including a spacer portion having first and second ends, the first end having a patient interface, at least one respiratory therapy device connected to the second end of the spacer portion, and a monitoring portion connected to the spacer portion; monitoring sounds within the apparatus using the monitoring portion; and delivering respiratory therapy from the respiratory therapy device, through the spacer portion, to the respiratory system of the patient.

It is yet another aspect of one or more embodiments to provide an apparatus for combined respiratory therapy delivery and respiratory monitoring, comprising: spacer means having first and second ends, the first end having a patient interface configured to engage a respiratory system of a patient; respiratory therapy means connected to the second end of the spacer means; and monitoring means connected to the spacer means configured to monitor sounds from within the apparatus.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.
FIG. 1 is an example of a combined respiratory therapy and respiratory monitoring apparatus, according to various embodiments of the invention.
FIG. 2 is an example of an analysis portion for a combined respiratory therapy and respiratory monitoring apparatus, according to various embodiments of the invention.
FIG. 3 is an example of a process for combined respiratory therapy and respiratory monitoring, according to various embodiments of the invention.
FIG. 4 is an example of time-frequency plots of respiratory sound recordings.
FIG. 5A is an example of respiratory sound processing for wheeze extraction, according to various embodiments of the invention.
FIG. 5B is an example of an input plot and a plot which indicates wheezes, according to various embodiments of the invention.
FIGS. 6A-6D are examples of combined respiratory therapy and respiratory monitoring apparatus, according to various embodiments of the invention.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The systems and methods described herein enable combined respiratory therapy and high-compliance respiratory monitoring. In some embodiments, the respiratory monitoring requires little to no effort or breathing maneuvers from a patient and provides an improved patient experience. In some embodiments, these systems and methods may be useful in treatment involving metered dose inhalers (MDIs) that may need to be used daily (or more or less frequently). In some embodiments, these systems may be useful in treatment involving ventilators or other flow or pressure based respiratory therapy devices. Combined respiratory therapy and respiratory monitoring can therefore obtain daily (or more or less frequent) data without any extra effort from the patient or clinician. As MDIs are commonplace among asthma sufferers, sound monitoring may be particularly advantageous for monitoring asthma indicators due to the fact that sound indicators (e.g., wheezes) are highly informative in asthma management.

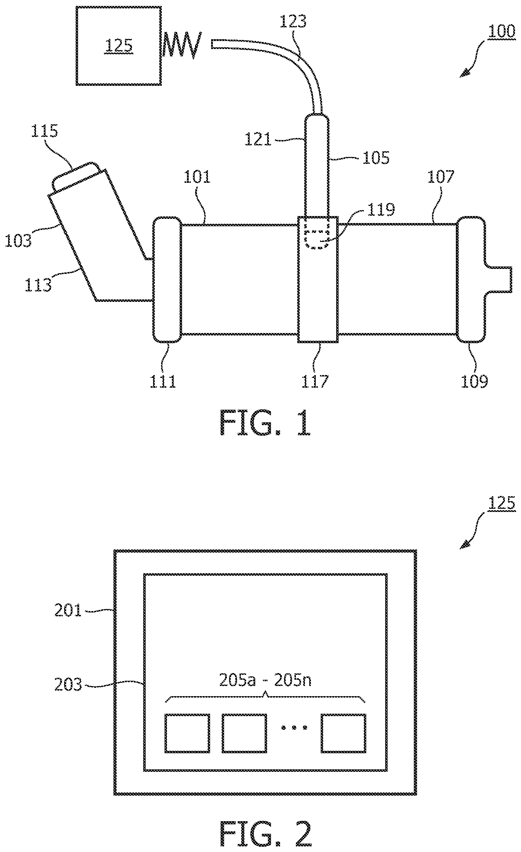

FIG. 1 illustrates an apparatus 100, which is an example of an apparatus for combined respiratory therapy and respiratory monitoring. In some embodiments, apparatus 100 includes a spacer portion 101, a respiratory therapy device 103, a monitoring device 105, and/or other elements.

In some embodiments, spacer portion 101 may separate respiratory therapy device 103 and a patient so as to better coordinate patient inhalation with respiratory therapy and/or to provide other features. In some embodiments, spacer portion 101 include body 107, which may have a first end and a second end. In some embodiments, body 107 may be or include a cylinder of plastic, glass, polymer, or other material. In some embodiments, body 107 may be or include a valved holding chamber. The valved holding chamber may have a valve therein for gating delivery of respiratory therapy to the patient and/or for providing other features.

In some embodiments, body 107 may include a patient interface 109 at a first end thereof. In some embodiments, patient interface 109 may be or include a mouthpiece, mask, tube, or other interface with the respiratory system of the patient. In some embodiments, body 107 may include a respiratory therapy device adapter 111 at a second end thereof. In some embodiments, respiratory therapy device adapter 111 may be or include a cover or cap for the second end of body 107, the cover or cap having an opening therein to receive an output of respiratory therapy device 103. In some embodiments, the opening is sized and/or includes flexible material (e.g., rubber) so as to create a seal with the output of respiratory therapy device 103 so that only material/gas flowing from respiratory therapy device 103 is delivered to spacer portion 101 and consequently to the patient.

In some embodiments, respiratory therapy device 103 may be or include a source of medicament/drug ("a drug source"). For example, in some embodiments, a drug source may include a metered dose inhaler (MDI) having a body 113 and a compressed source of drug 115. Body 113 may include an aerosolizer/nebulizer/atomizer (not illustrated) through which drug is aerosolized when the drug is released from compressed source of drug 115 (e.g., via a pressure-based release valve - not illustrated). Examples of MDI's that may be used as a respiratory therapy device 103 may include inhalers typically used to treat asthma in adults and children. In some embodiments, an inhaler used as respiratory therapy device 103 may include a mouthpiece that is, in some instances, used by a patient to interface with the patient's mouth/respiratory system. In some embodiments, this mouthpiece may be the output of respiratory therapy device 103 that fits into a sized opening of drug source adapter 111. In some embodiments, drug sources other than MDI's may be used (e.g., dry powder inhalers, nebulizers, etc.).

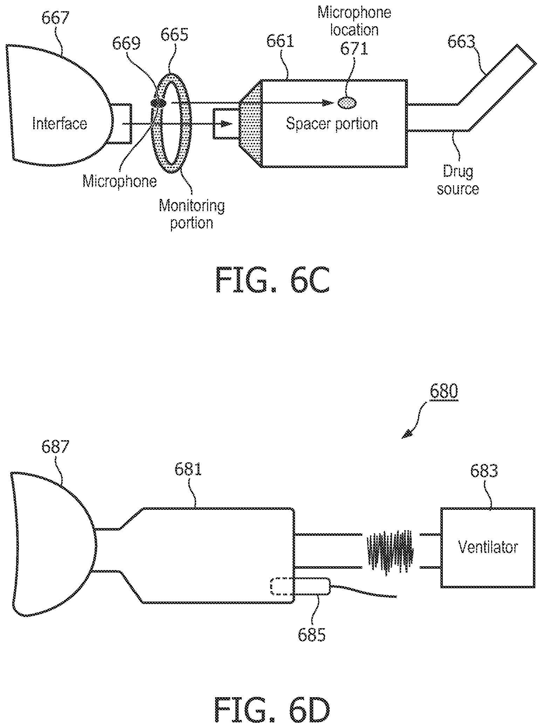

In some embodiments, a respiratory therapy device used with the systems and methods described herein may be or include a device that provides a flow of gas to the respiratory system of a patient. For example, the respiratory therapy device may be a ventilator or respiratory device used in the treatment of numerous ailments/conditions (see e.g., FIG. 6D). In some embodiments, the respiratory therapy device may include a positive airway pressure (PAP) device.

In some embodiments, monitoring device 105 may be or include a microphone or other sound receiving device. For example, in some embodiments, monitoring device 105 may include a sound receiving portion 119, a body 121, one or more connections 123 and/or other elements. Sound receiving portion 119 may include a microphone tip that receives the sounds from within body 107 of spacer portion 101. These sounds may, for example, include respiratory sounds of a patient using apparatus 100 (e.g., inhalation sounds, exhalation sounds, wheezes, crackles, or other respiratory sounds). These sounds may also include sounds relating to delivery of respiratory therapy such as, for example, sounds relating to gas flow within spacer portion 101 (which may be caused by respiration of the patient, by a respiratory therapy device and/or other sources), the firing or other operation of a respiratory therapy device (e.g., the firing of a drug delivery device, operation of components of a ventilator, or other operating sounds of a respiratory therapy device), and/or other sounds.

In some embodiments, to receive these sounds, sound receiving portion 119 may be disposed within body 107 of spacer portion 101. In some embodiments, this positioning may be facilitated by an adapter 117, which may be or include a collar, ring (e.g., rigid, elastic) or other device that is attached to body 107 and holds body 121 of monitoring device 105 to body 107 of spacer portion 101. While illustrated in FIG. 1 as disposed within body 107 of spacer portion 101, monitoring device 105 may be connected to patient interface 109, drug source adapter 111, and/or otherwise attached so as to sample a patient's respiratory sounds or sounds from within an apparatus for combined respiratory therapy and respiratory monitoring.

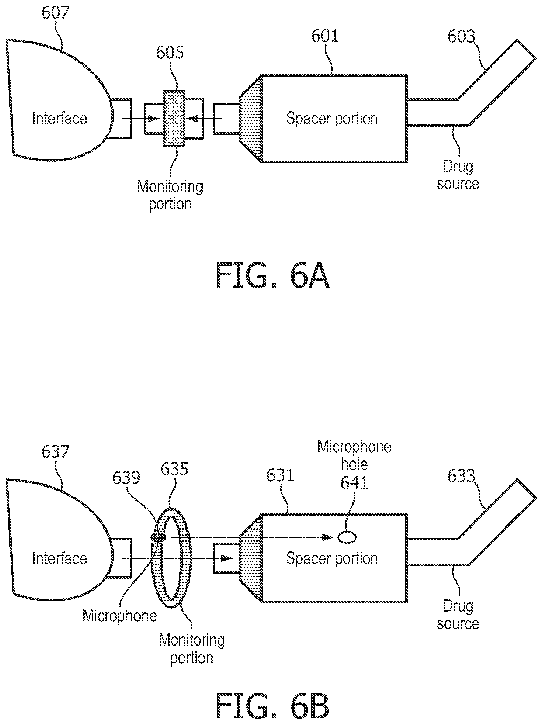

In some embodiments, placement of a monitoring device may be made so as to not (or minimally) obstruct provision of therapy (e.g., a flow of gas or drug) to the patient. For example, FIG. 1 illustrates that sound receiving portion 119 is positioned in the upper portion of spacer portion 101 so as to avoid interfering with delivery of therapy. FIGS. 6B-6C illustrate placement of a monitoring device over a hole or designated portion of a spacer portion so that the monitoring device does not protrude (or minimally protrudes) into the body of a spacer portion of the device. FIG. 6D illustrates that a monitoring portion 685 may be located at the back of a spacer portion 681 of the device (e.g., adjacent to the inlet of respiratory therapy device 683 and opposite the end of spacer portion 681 closest to patient interface 687). Other placements of a monitoring device may be used.

The sounds received/monitored by sound receiving portion 119 may be transmitted to an analysis portion 125 (e.g., via connections 123) to be stored and/or analyzed. FIG. 2 illustrates an example of an analysis portion 125 that maybe used with apparatus 100. Analysis portion 125 maybe or include one or more computing devices 201 that provide storage, analysis, and or output related to the sounds monitored by monitoring device 105. One or more computing devices 201 of analysis portion 125 may include one or more data processing devices (e.g., microprocessors), volatile and/or nonvolatile memory devices (e.g., hard disks, RAM, etc.), communication interfaces, input/output devices, and/or other elements. In some implementations, one or more computing devices 201 may run, execute, or host an analysis application 203, which may be or include a software application having one or more modules 205a-205n. Modules 205a-205n may include instructions for causing the one or more processors of one or more computing devices 201 to perform one or more of features and functions relating to respiratory and/or other sound analysis such as, for example, receiving sounds from within device 100 (e.g., from monitoring device 105), storing received sounds, analyzing/filtering/processing received sounds, producing one or more outputs based at least partially on received sounds (e.g., making diagnosis or treatment determinations), and/or other features and functions.

In some embodiments, one or more computing devices 201 may receive and send information through wired connections (e.g., connections 123), through wireless connections, and/or a combination of both. In some embodiments, one or more computing devices 201 may receive and send information or otherwise be connected to one ore more computer networks such as, for example, a local area network (LAN), a wide area network (WAN), an intranet, the Internet, and/or other computing networks.

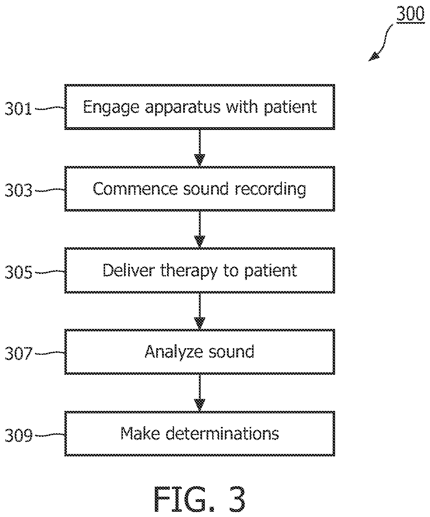

FIG. 3 illustrates a process 300, which is a process for providing combined respiratory therapy and respiratory monitoring. Process 300 may include an operation 301, wherein an apparatus for combined respiratory therapy and respiratory monitoring (e.g., apparatus 100, 600, 630, 660, 680, or other device according to the descriptions provided herein) is engaged with a patient. In some embodiments, this may include the patient placing a patient interface (e.g., patient interface 109) in (e.g., when the interface is a mouthpiece) or over (e.g., when the interface is a mask) the patient's mouth. Other methods of engaging an apparatus for combined drug delivery and respiratory monitoring to a patient may be used (e.g., via trans-tracheal tube, via intubation, etc.).

In an operation 303, monitoring, by the apparatus (e.g., monitoring device 105 of apparatus 100), of the patient's respiratory sounds or other sounds within the apparatus, may commence. This monitoring may include recording/detecting breath and other respiratory sounds of the patient such as, for example, inhalation sounds, exhalation sounds, wheezes, crackles, and/or other respiratory sounds. This monitoring may also include recording/detecting other sounds such as, flow related sounds within the apparatus, operation of respiratory therapy devices, or other sounds. The monitored sounds may be recorded/stored via a computer implemented device (e.g., computing device 201 of analysis portion 125), on an analog sound recording device (e.g., tape recorder), digital sound recording device (e.g., digital voice recorder), or otherwise stored. In some embodiments, monitoring of sounds may occur for a predetermined period of time or may occur indefinitely.

Monitoring of operation 303 may occur before, during, and/or after performance of respiratory treatment or may be independent of respiratory treatment. The duration of monitoring may be determined based on what is needed for a specific analysis (e.g., one respiration cycle, 2 cycles, 3 cycles, or more). In some embodiments, monitoring events may be conducted once a day. In some embodiments, monitoring events may be conducted more or less frequently, as desired or necessary.

In an operation 305, respiratory treatment may be delivered from the apparatus. For example, drugs or other therapeutic substances may be delivered from the apparatus to the patient. If a metered dose inhaler is attached to or part of the respiratory therapy and respiratory monitoring apparatus, then the patient, medical professional, or other person may actuate the metered dose inhaler so that drug is released to the patient. The patient may inhale the released drug, thereby delivering it to the respiratory system. An example of the drug delivered may include any one or more of a number of bronchodilators, for example, those used to treat asthma. Other drugs may also be used.

As described herein, while apparatus 103 is illustrated as a drug delivery apparatus (an MDI), the treatment provided in operation 305 need not be drug delivery. For example, in some embodiments, the apparatus may include a ventilator or other respiratory therapy device providing a flow of air to the patient. In some embodiments, the respiratory sounds detected/recorded are not limited to those associated with asthma or COPD, but may be those providing information relating to pneumonia or other respiratory conditions.

In an operation 307, the monitored sounds may be analyzed. In some embodiments, this operation may include the monitored sounds being received by an analysis portion of an apparatus for combined respiratory therapy and respiratory monitoring (e.g., analysis portion 125 of apparatus 100). The analysis portion may include a data reception module (e.g., one of modules 205a-205n) for receiving sound data from a monitoring portion (monitoring portion 105).

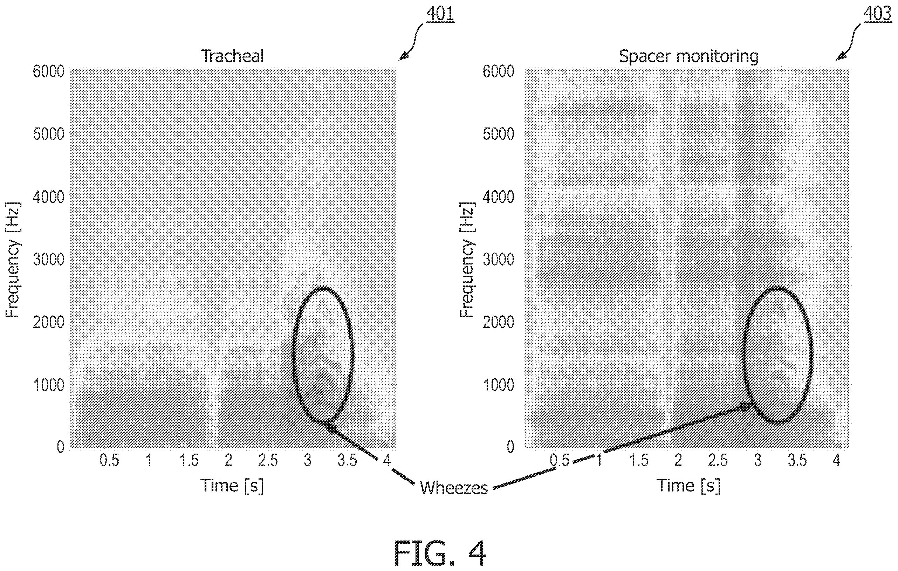

In one example of analysis performed in operation 307, wheeze sounds may be extracted from the monitored sounds for determinations of the state of an asthmatic patient's respiratory system. FIG. 4 illustrates sound recordings of patient respiratory sounds (plotted in frequency vs. time). Recording 401 illustrates a recording of respiratory sounds using a tracheal setup and recording 403 illustrates a recording of the same respiratory sounds taken from a spacer of a respiratory therapy apparatus. Wheezes are apparent in a time-frequency plot as high intensity curves with narrowband and usually frequency-varying character, as is evident in FIG 4. Prevailing wisdom has been that tracheal-based sound monitoring is necessary for certain monitoring (e.g., wheeze detection). However, FIG. 4 illustrates that wheezes produced by a patient can be detected using a spacer mounted monitor and therefore that a separate, tracheal placed monitor is not necessary. While recording 403 includes a worse signal to noise ratio, the wheeze is still clearly visible, therefore displaying the viability of drug-delivery-apparatus-based monitoring.

Because of the presence of the normal wideband breathing sound and the aforementioned presence of noise in a spacer-based recording, certain processing may be performed to enhance wheeze (or other sound) detection. Processes and procedures used to detect wheezes may be similarly adapted to detect crackles. The exemplary process described herein is based on a combination of conventional one-dimensional filtering followed by the application of image processing techniques to a spectrogram of a respiratory sound recording.

The fact that wheezes exhibit non-vertical narrowband edges in the time-frequency plane (i.e. they possess a musical character) can be used for detection. These non-vertical narrowband edges make wheezes distinguishable from background wideband breathing sound. Crackles exhibit (near-)vertical narrowband edges in the time-frequency plane, which also makes them distinguishable from the background wideband breathing sound. Accordingly, these (near-)vertical narrowband edges can be used to distinguish crackles from background breathing sound.

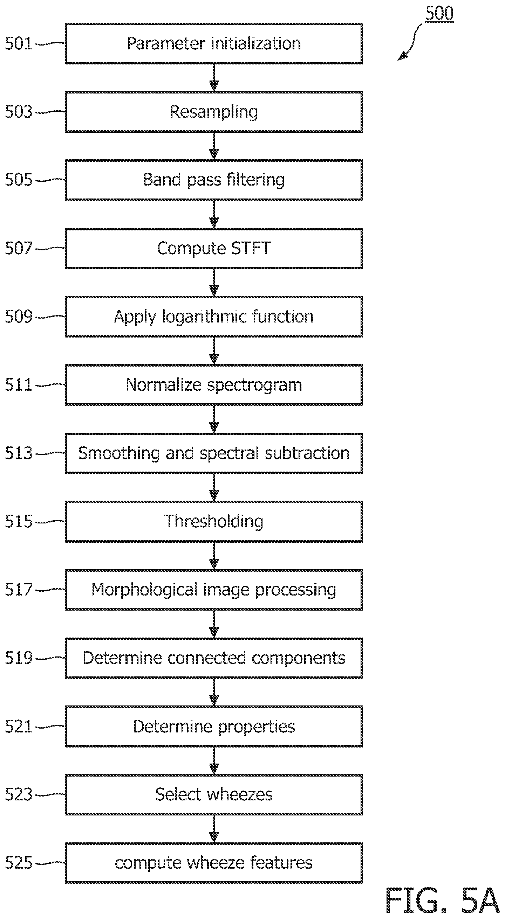

FIG. 5A provides an example wheeze detection process 500. However, as discussed above, modified processes may be used to detect other respiratory sounds such as, for example, crackles or other respiratory sounds. Additionally, other processes may be used to detect wheezes or other respiratory sounds.

Process 500 may include an initialization operation 501, wherein various parameters are specified. This may include parameters related to spectral analysis such the fast fourier transform (FFT) length used for computing the Short-Time Fourier Transform (STFT), the window used for tapering during STFT computation, the overlap between windows, and so on. In addition, cut-off frequencies of the employed band-pass filter (BPF), some thresholds, and some criteria for wheeze detections may also be specified.

In an operation 503, recorded data samples may be resampled for further analysis. For example, in some instances, recorded data samples are sampled with a frequency of, for example, 44100 Hz. These samples may be resampled to 11025 Hz or other frequency convenient for further processing.

In an operation 505, a band-pass filter (BPF) may be applied to the samples to filter out low and high frequencies. This is done because it is known that (almost) no wheeze is present at such frequencies. Accordingly, the PBF is used to get rid of noise and other undesired spectral contents.

In an operation 507, a Short-Time Fourier Transform (STFT) may be computed. In some instances, only the magnitude of the STFT is used. This is usually referred to as the spectrogram. Plots of the spectrogram clearly exhibit wheezes (if present), while plots of the phase of the STFT do not. The parts of the time-frequency plane where the spectrogram has a certain absolute magnitude are not of particular interest. Rather, the narrowband signal components that rise above the local breathing background are of more interest. For this reason, the dynamic range may be limited in an operation 509 by applying a logarithmic (dB) function.

In order to make the subsequent analysis as independent as possible from the chosen parameter values, and to reduce the effects of different recording conditions and different subjects, the spectrogram may be normalized in an operation 511. In particular, the spectrogram expressed in dB's (i.e., from application of the logarithmic function in operation 509) is translated and scaled in such a way that its minimum and maximum are given by 0 and 1 respectively.

In order to get rid of noise and breathing background smoothing and spectral subtraction may be performed in an operation 513. The spectrogram/image resulting from operation 513 may then be converted to Black-White (BW) by means of thresholding in an operation 515. Effectively, an image with the logical values 0 and 1 is obtained, where the ones indicate wheezing candidates.

In an operation 517, the result of operation 515 may be subjected to morphological image processing operations such as image opening, closing, dilation and erosion. In some instances, only (binary) image opening may be used.

In an operation 519, image components whose pixels are connected are determined. In an operation 521, one or more properties of each component such as, for example, area, maximum width (representing duration), maximum height (representing frequency variation), and/or other properties are computed.

In an operation 523, connected components having certain properties are selected as wheezes. For example, the area of a component is required to lie in a certain interval and the duration and frequency variation of each component must be larger and smaller respectively than certain bounds.

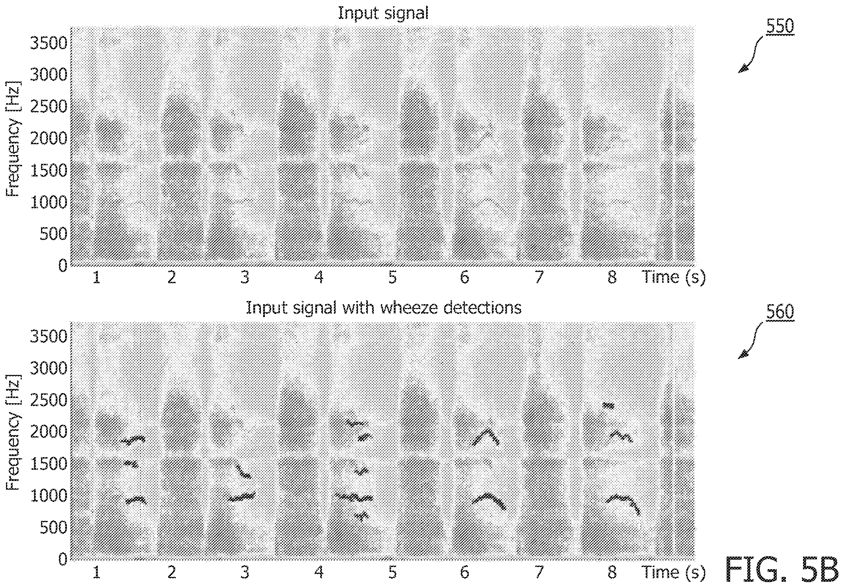

Finally, in an operation 525, wheeze features such as, for example, duration and frequency variation are computed for each connected component. These may be used be used to define an overall measure of wheezing severity such as, for example, the percentage of time in which a patient is wheezing. FIG. 5B illustrates plots 550 and 560, which show an example of the application of the algorithm to recorded data. Plot 550 illustrates the input signal, while plot 560 illustrates the input signal with identified/detected wheezes.

In some implementations, extracted wheeze sounds may be used to determine a percentage of time in which a patient is wheezing, evaluate the intensity of wheezes, the frequency of wheezes, and/or other metrics for the patient. These characteristics may be used to monitor the state of a patient's respiratory system, adjust treatment parameters, and/or for other purposes. For example, respiratory sounds may be used to reflect a level of asthma control. In some embodiments, such level of control or other use of sound data may be displayed visually to users via a display such as, for example a series of lights, a textual display, or other display. In some implementations, sound data may be used as input for determining medication levels (e.g., step-up/step-down scenarios). In some embodiments, data may be stored and communicated to a medical professional or other person so as to enable evaluation of trends over a long term period of time so that treatment can be altered accordingly.

While the above sound processing is described with respect to extracting wheeze sounds, those having skill in the art would recognize that similar operations may be used to process/extract crackle or other sounds as well. Crackle sounds from the respiratory system of a patient may be indicative of chronic obstructive pulmonary disease (COPD). Other types of sounds may be processed/extracted from respiratory sounds for use in conjunction with asthma, COPD, and/or other respiratory ailments.

Any processing (e.g., de-noising, de-convoluting) and/or other analysis (e.g., wheeze detection) may be performed by one or more modules of an analysis portion of an apparatus for combined drug delivery and respiratory monitoring (e.g., one or more of modules 205a-205n of analysis portion 125) specifically programmed for such processing and analysis. The above wheeze extraction process is an example only. Other analysis may be performed on sound data from an apparatus for combined respiratory therapy and respiratory monitoring, including detection of breathing patters or other breathing characteristics such as, for example, the presence of crackles (e.g., for COPD patients). In some embodiments, processing steps may be performed in a different order than the examples given herein.

Analysis of monitored sounds can also be used to determine (e.g., by computer-implemented analysis portion 125) whether a respiratory treatment device is effectively delivering the intended respiratory therapy to the respiratory system of the patient. Based on this determination, one or more recommendations or instructions maybe formulated (e.g., by medical personnel or by analysis portion 125) to correct the use of the respiratory therapy device so as to ensure that respiratory therapy is effectively delivering the intended respiratory therapy to the patient. For example, firing of a drug delivery burst from a respiratory therapy device, shaking sounds, and/or other sounds can be detected and analyzed to make one or more determinations relating to the effectiveness of respiratory treatment. For example, the time between firing of a drug source and inhalation (which can be detected using respiratory sounds) should not exceed a certain calculated threshold. If such a threshold is exceeded, the timing of firing of a drug source can be adjusted. In another example, the amount of noise during inhalation or exhalation can be analyzed to determine the strength of airflow and whether such flow needs to be adjusted. For example, based on this flow, timing of medication/drug delivery may be adjusted (e.g., if inhalation flow is too high, drug may be delivered to the throat rather than the lungs, which is undesirable; therefore, flow may be adjusted based on this flow estimate so that drug is properly delivered to the lungs).

Returning to FIG. 3, in an operation 309, one or more determinations may be made using the analyzed/processed data and/or other data. For example, as discussed herein, treatment for patients may be formulated or altered based on the analyzed data (e.g., wheeze rate may lead to an increase or decrease in dispensed drug to the patient). In another example a state of a patient or diagnosis may be determined using the analyzed data (e.g., wheeze rate may be used to diagnose a patient with asthma). These determinations may be made by medical professionals or other personnel, or may be made (or assisted) by one or more computer-implemented modules (e.g., modules 205a-205n) having rules or decision-making logic therein facilitating such decisions (e.g., intense/more-frequent wheezes = higher doses or more frequent doses of drug). In one example, a determination made by one or more computer-implemented modules may include a suggestion to see a medical professional (e.g., due to detection of a worsening condition). In some embodiments, these determinations may be based on a single measurement of respiratory sound. However, in some embodiments, these determinations may be made using trends in sound sampling. For example, if an amount of wheezing is increasing rapidly, a determination to see a doctor may be made. In another example, if wheezing is detected, but it is improving, a determination that no doctor visit is needed may be made.

The systems described herein are exemplary system configurations. Other configurations may exist. The components of the example systems illustrated and described herein may be mixed and matched as would be appreciated by those of ordinary skill in the art.

As mentioned herein, the placement of a monitoring device in a drug delivery apparatus may introduce additional noise into respiratory recordings (see e.g., FIG. 4). Airflow in a spacer portion may contribute to this noise. Accordingly, design of the chamber provided by the spacer portion and the placement of the monitoring device (e.g., microphone) therein may be manipulated to reduce such noise as much as possible. For example, the size and material of the spacer portion may be chosen such that no resonance or evident coloring appears at those frequencies in which wheezes (or other significant breath sounds) are expected (for wheezes, the 200-1200Hz band). Moreover, integration of the monitoring device into the patient interface (e.g., a mask or mouthpiece) or between the interface and the spacer portion may be used. Also, for practical reasons (e.g., hygiene), it may be desirable to implement a monitoring portion that is not fully embedded into the apparatus, but as an add-on or snap-on module. This may enable removal of electronic components during cleaning for re-use of the delivery apparatus. Also, if the relatively low cost delivery apparatus needs to be discarded, the higher cost electronic components may be re-used with a new delivery apparatus. As such, the monitoring portion may be part of a removable device that can be integrated with a drug delivery apparatus.

FIGS. 6A-6D illustrate examples of some varying configurations of a combined respiratory therapy and respiratory monitoring apparatus. FIG. 6A illustrates a combined respiratory therapy and respiratory monitoring apparatus 600, which includes a spacer portion 601, a respiratory therapy device 603, a monitoring portion 605, and a patient interface 607. As illustrated, monitoring portion 605 is positioned between patient interface 607 and spacer portion 601.

FIG. 6B illustrates a combined respiratory therapy and respiratory monitoring apparatus 630, which includes a spacer portion 631, a respiratory therapy device 633, a monitoring portion 635, and a patient interface 637. As illustrated, monitoring portion 635 may be a ring (e.g., rigid ring, elastic band, etc.) that includes a microphone 639. The ring can be slid over spacer portion 631 such that microphone 639 is positioned over microphone hole 641.

FIG. 6C illustrates a combined respiratory therapy and respiratory monitoring apparatus 660, which includes a spacer portion 661, a respiratory therapy device 663, a monitoring portion 665, and a patient interface 667. Similar to apparatus 630, monitoring portion may be a ring that includes a microphone 669 that is slid over spacer portion 661. However, rather than a hole for microphone 669, spacer portion 661 may include a microphone location 671 that is of a different thickness than the remainder of spacer portion 661 or is made of a different material for the purposes of better capturing respiratory sound.

FIG. 6D illustrates a combined respiratory therapy and respiratory monitoring apparatus 680, which includes a spacer portion 681, a respiratory therapy device 683, a monitoring portion 685, and a patient interface 687. As illustrated in FIG. 6D, monitoring device 685 is located at the back of spacer portion 681 so as not to interfere with respiratory therapy. In this manner, monitoring device 685 is located generally out of the pathway of a flow associated with respiratory therapy (e.g., a flow of drug and/or a flow of air, etc.). As illustrated in FIG. 6D, respiratory therapy device 683 is a ventilator. However, as discussed herein, a drug source (e.g., MDI) or other respiratory therapy device may be used. Location of a monitoring device at the back of a spacer portion is not limited to use with respirators as therapy devices, but can also be used with drug sources or other therapy devices.

Those having skill in the art will appreciate that the invention described herein may work with various configurations. Accordingly, more or less of the aforementioned system components may be used and/or combined in various embodiments. It should also be understood that various software modules that are utilized to accomplish the functionalities described herein may be maintained on different components than computer-implemented device 201, as desired or necessary. In other embodiments, as would be appreciated, the functionalities described herein may be implemented in various combinations of hardware and/or firmware, in addition to, or instead of, software. Furthermore, various operations of the methods described herein, while described in a particular order, may be performed in different orders as would be appreciated by those having skill in the art. In some embodiments, more of less of the described operations may be used.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. An apparatus for combined respiratory therapy and respiratory monitoring, comprising:
a patient interface (109) configured to engage a respiratory system of a patient;
at least one respiratory therapy device (103, 683);
a computer-implemented analysis portion (125) for determination of a condition of the respiratory system of the patient; and
a monitoring portion (105) configured to monitor sounds from within the apparatus wherein the monitoring portion (105) includes a microphone that provides the monitored sounds to the computer-implemented analysis portion; **characterised by**:
a spacer portion (101) having first and second ends, the first end having the patient interface (109), the second end connected to the at least one respiratory therapy device (103, 683), and the monitoring portion (105) connected to the spacer portion (101); wherein the computer implemented analysis portion (125) is configured to extract one or more of wheeze sounds or crackle sounds from the monitored sounds.

2. The apparatus of claim 1, wherein the at least one respiratory therapy device (103) includes a drug source that, when actuated, delivers a dose of a drug through the spacer portion to the respiratory system of the patient when the respiratory system of the patient is engaged to the patient interface.

3. The apparatus of claim 1, wherein the at least one respiratory therapy device includes a ventilator (683) that delivers a pressurized flow of air through the spacer portion to the respiratory system of the patient when the respiratory system of the patient is engaged to the patient interface.

4. The apparatus of claim 1, wherein the computer-implemented analysis portion (125) is further configured to determine, using the monitored sounds, whether the respiratory therapy device (103, 683) is effectively delivering therapy to the respiratory system of the patient.

5. The apparatus of any preceding claim wherein the monitoring portion (105) is placed so as to minimise obstructing provision of therapy to the patient.

6. The apparatus of claim 5 wherein the monitoring portion (105) is placed so as to minimise protrusion into a body (107) of the spacer portion (101).

7. The apparatus of claim 6 wherein the monitoring portion (105) is provided in an adapter which holds the monitoring portion to the body (107) of the spacer portion (101).

8. The apparatus of claim 6 wherein the adapter comprises a ring which can be slid over the spacer portion (101).

9. A method of operating an apparatus as claimed in claim 1 for respiratory monitoring independent of respiratory therapy, the method comprising:
- monitoring sounds from within the apparatus using the microphone;
- providing the monitored sounds to the computer-implemented analysis portion (125); and
- determining, at the computer implemented analysis portion (125), a condition of the respiratory system of the patient using the monitored sounds, and wherein determining a condition of the respiratory system of the patient includes extracting one or more of wheeze sounds or crackle sounds from the monitored sounds.

## Patentansprüche

1. Vorrichtung für eine kombinierte Atemtherapie und Atmungsüberwachung, umfassend:
eine Patientenschnittstelle (109), die dafür ausgelegt ist, an einem Atemsystem eines Patienten verbunden zu werden;
mindestens ein Atemtherapiegerät (103, 683); einen computerimplementierten Analyseabschnitt (125) für eine Bestimmung eines Zustands des Atemsystems des Patienten; und
einen Überwachungsabschnitt (105), der dafür ausgelegt ist, Geräusche aus dem Inneren der Vorrichtung zu überwachen, wobei der Überwachungsabschnitt (105) ein Mikrofon aufweist, das die überwachten Geräusche an dem computerimplementierten Analyseabschnitt bereitstellt; **gekennzeichnet durch**:
einen Abstandhalterabschnitt (101) mit einem ersten und einem zweiten, die Patientenschnittstelle (109) aufweisenden Ende, wobei das zweite Ende an dem mindestens einen Atemtherapiegerät (103, 683) angeschlossen ist und der Überwachungsabschnitt (105) an dem Abstandhalterabschnitt (101) angeschlossen ist;
wobei der computerimplementierte Analyseabschnitt (125) dafür ausgelegt ist, pfeifende Geräusche und/oder rasselnde Geräusche aus den überwachten Geräuschen zu extrahieren.

2. Vorrichtung nach Anspruch 1, wobei das mindestens eine Atemtherapiegerät (103) eine Arzneiquelle aufweist, die, wenn sie betätigt wird, eine Dosis eines Arzneimittels durch den Abstandhalter hindurch an das Atemsystem des Patienten abgibt, wenn das Atemsystem des Patienten mit der Patientenschnittstelle verbunden ist.

3. Vorrichtung nach Anspruch 1, wobei das mindestens eine Atemtherapiegerät einen Ventilator (683) aufweist, der einen Strom von unter Druck gesetzter Luft durch den Abstandhalterabschnitt hindurch an das Atemsystem des Patienten abgibt, wenn das Atemsystem des Patienten mit der Patientenschnittstelle verbunden ist.

4. Vorrichtung nach Anspruch 1, wobei der computerimplementierte Analyseabschnitt (125) ferner dafür ausgelegt ist, unter Verwendung der überwachten Geräusche zu bestimmen, ob das Atemtherapiegerät (103, 683) auf effektive Weise eine Therapie an das Atemsystem des Patienten abgibt.

5. Vorrichtung nach einem der vorangehenden Ansprüche, wobei der Überwachungsabschnitt (105) so platziert ist, dass er eine Bereitstellung einer Therapie für den Patienten so wenig wie möglich behindert.

6. Vorrichtung nach Anspruch 5, wobei der Überwachungsabschnitt (105) so platziert ist, dass er so wenig wie möglich in einen Körper (107) des Abstandhalterabschnitts (101) vorsteht

7. Vorrichtung nach Anspruch 6, wobei der Überwachungsabschnitt (105) in einem Adapter bereitgestellt ist, der den Überwachungsabschnitt am Körper (107) des Abstandhalterabschnitts (101) hält.

8. Vorrichtung nach Anspruch 6, wobei der Adapter einen Ring umfasst, der über den Abstandhalterabschnitt (101) geschoben werden kann.

9. Verfahren zum Betätigen einer Vorrichtung nach Anspruch 1 für eine von einer Atemtherapie unabhängige Atmungsüberwachung, wobei das Verfahren umfasst:
- Überwachen von Geräuschen aus dem Inneren der Vorrichtung unter Verwendung des Mikrofons;
- Bereitstellen der überwachten Geräusche an dem computerimplementierten Analyseabschnitt (125); und
- Bestimmen eines Zustands des Atemsystems des Patienten an dem computerimplementierten Analyseabschnitt (125) unter Verwendung der überwachten Geräusche, und wobei die Bestimmung eines Zustands des Atemsystems des Patienten das Extrahieren von pfeifenden Geräuschen und/oder rasselnden Geräuschen aus den überwachten Geräuschen einschließt.

## Revendications

1. Appareil de thérapie respiratoire et de surveillance respiratoire combinées, comprenant :
une interface patient (109) configurée pour engager un système respiratoire d'un patient ; au moins un dispositif de thérapie respiratoire (103, 683) ;
une partie d'analyse mise en œuvre par ordinateur (125) pour déterminer un état du système respiratoire du patient ; et
une partie de surveillance (105) configurée pour surveiller des sons de l'intérieur de l'appareil, dans lequel la partie de surveillance (105) comprend un microphone, lequel fournit les sons surveillés à la partie d'analyse mise en œuvre par ordinateur ; **caractérisé par** :
une partie d'espacement (101) comportant des première et seconde extrémités, la première extrémité comportant l'interface patient (109), la seconde extrémité connectée à l'au moins un appareil de thérapie respiratoire (103, 683) et la partie de surveillance (105) connectée à la partie d'espacement (101) ;
dans lequel la partie d'analyse mise en œuvre par ordinateur (125) est configurée pour extraire au moins un son sifflant ou grésillements à partir des sons surveillés.

2. Appareil selon la revendication 1, dans lequel l'au moins un appareil de thérapie respiratoire (103) comprend une source de médicament, laquelle, lorsqu'elle est actionnée, administre une dose d'un médicament à travers la partie d'espacement au système respiratoire du patient lorsque le système respiratoire du patient est en prise avec l'interface patient.

3. Appareil selon la revendication 1, dans lequel l'au moins un appareil de thérapie respiratoire comprend un ventilateur (683), lequel administre un flux d'air sous pression à travers la partie d'espacement vers le système respiratoire du patient lorsque le système respiratoire du patient est en prise avec l'interface patient.

4. Appareil selon la revendication 1, dans lequel la partie d'analyse mise en œuvre par ordinateur (125) est en outre configurée pour déterminer, à l'aide des sons surveillés, si le dispositif de thérapie respiratoire (103, 683) administre effectivement une thérapie au système respiratoire du patient.

5. Appareil selon l'une quelconque des revendications précédentes, dans lequel la partie de surveillance (105) est placée de manière à minimiser l'obstruction de la fourniture d'une thérapie au patient.

6. Appareil selon la revendication 5, dans lequel la partie de surveillance (105) est placée de manière à minimiser une saillie dans un corps (107) de la partie d'espacement (101).

7. Appareil selon la revendication 6, dans lequel la partie de surveillance (105) est située dans un adaptateur, lequel maintient la partie de surveillance sur le corps (107) de la partie d'espacement (101).

8. Appareil selon la revendication 6, dans lequel l'adaptateur comprend un anneau, lequel peut être glissé sur la partie d'espacement (101).

9. Procédé de fonctionnement d'un appareil selon la revendication 1 destiné à la surveillance respiratoire indépendante de la thérapie respiratoire, ledit procédé comprenant :
la surveillance des sons provenant de l'intérieur de l'appareil à l'aide du microphone ;
la fourniture des sons surveillés à la partie d'analyse mise en œuvre par ordinateur (125) ; et
la détermination, au niveau de la partie d'analyse mise en œuvre par ordinateur (125), d'un état du système respiratoire du patient à l'aide des sons surveillés, et dans lequel la détermination d'un état du système respiratoire du patient comprend l'extraction d'au moins un son sifflant ou grésillement à partir des sons surveillés.
